(19) Europäisches Patentamt
European Patent Office
Office européen des brevets

(11) EP 1 406 606 B1

(12) EUROPEAN PATENT SPECIFICATION

(45) Date of publication and mention of the grant of the patent:
**10.01.2007 Bulletin 2007/02**

(21) Application number: **02752248.1**

(22) Date of filing: **11.07.2002**

(51) Int Cl.:
***A61K 31/325*** *(2006.01)* ***A61K 31/27*** *(2006.01)*
***A61P 25/06*** *(2006.01)*

(86) International application number:
**PCT/US2002/021787**

(87) International publication number:
**WO 2003/007936 (30.01.2003 Gazette 2003/05)**

# (54) CARBAMATE COMPOUNDS FOR USE IN PREVENTING OR TREATING NEUROPATHIC PAIN AND CLUSTER AND MIGRAINE HEADACHE-ASSOCIATED PAIN

VERWENDUNG VON CARBAMAT-VERBINDUNGEN ZUR PRÄVENTION ODER BEHANDLUNG VON NEUROPATHISCHEN SCHMERZEN UND MIGRÄNEARTIGEN KOPFSCHMERZEN

COMPOSES DE CARBAMATE DESTINE A ETRE UTILISES DANS LA PREVENTION OU LE TRAITEMENT DE LA DOULEUR NEUROPATHIQUE ET DE LA DOULEUR ASSOCIEE A LA CEPHALEE VASCULAIRE DE HORTON ET A LA MIGRAINE

(84) Designated Contracting States:
**AT BE BG CH CY CZ DE DK EE ES FI FR GB GR IE IT LI LU MC NL PT SE SK TR**

(30) Priority: **16.07.2001 US 305687 P**
**11.07.2002 US 192973**

(43) Date of publication of application:
**14.04.2004 Bulletin 2004/16**

(73) Proprietor: **Ortho-McNeil Pharmaceutical, Inc.**
**Raritan, NJ 08869-0602 (US)**

(72) Inventors:
- **CODD, Ellen, C.**
  **Blue Bell, PA 19422 (US)**
- **PLATA-SALAMAN, Carlos, R.**
  **Amble, PA 19002 (US)**
- **ZHAO, Boyu**
  **Lansdale, PA 19446 (US)**

(74) Representative: **Fisher, Adrian John et al**
**CARPMAELS & RANSFORD**
**43-45 Bloomsbury Square**
**London WC1A 2RA (GB)**

(56) References cited:
**WO-A-02/07822** **US-A- 6 103 759**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Cross Reference to Related Application

This application claims priority from United States provisional application Serial No. 60/305,687 filed July 16, 2001.

Field of the Invention

This invention is directed to a method for use of a carbamate enantiomer in preventing or treating neuropathic pain and cluster and migraine headache-associated pain. More particularly, this invention is directed to a method for use of a halogenated 2-phenyl-1,2-ethanediol monocarbamate enantiomer substantially free of other enantiomers for preventing or treating neuropathic pain and cluster and migraine headache-associated pain.

Background of the Invention

The conditions grouped under the term neuropathic pain constitute an area of continuing medical need.

Neuropathic pain is defined as pain caused by aberrant somatosensory processing in the peripheral or central nervous system and includes painful diabetic peripheral neuropathy, post-herpetic neuralgia, trigeminal neuralgia, post-stroke pain, multiple sclerosis-associated pain, neuropathies-associated pain such as in idiopathic or post-traumatic neuropathy and mononeuritis, HIV-associated neuropathic pain, cancer-associated neuropathic pain, carpal tunnel-associated neuropathic pain, spinal cord injury-associated pain, complex regional pain syndrome, fibromyalgia-associated neuropathic pain, lumbar and cervical pain, reflex sympathic dystrophy, phantom limb syndrome and other chronic and debilitating condition-associated pain syndromes.

Cluster headache (also called Raeder's syndrome, histamine cephalalgia and sphenopalatine neuralgia) is characterized by a series of short-lived attacks of periorbital pain on an almost daily basis over a relatively short period of time (for example, over 4 to 8 weeks) followed by a pain-free interval. Migraine headache is also a periodic recurring disorder that can be associated with paroxysmal pain, vomiting, and photophobia. Migraine headaches include, and are not limited to, classic migraine (migraine with aura: associated with premonitory sensory, motor or visual symptoms) and common migraine (migraine without aura). Cluster and migraine headache-associated pain are also clinical indications with significant unmet medical need.

Neuropathic pain, migraine and cluster headache are all associated with changes in neuronal excitability (Mulleners W.M., et al, Visual Cortex Excitability in Migraine With and Without Aura, *Headache,* **2001**, Jun, 41 (6), 565-572; Aurora S.K., et al, The occipital cortex is hyperexcitable in migraine: experimental evidence, *Headache,* **1999,** Jul-Aug, 39(7), 469-76; Brau M.E., et al, Effect of drugs used for neuropathic pain management on tetrodotoxin-resistant Na(+) currents in rat sensory neurons, *Anesthesiology,* **2001,** Jan, 94(1), 137-44; Siddall P.J. and Loeser J.D., Pain following spinal cord injury, *Spinal Cord,* **2001,** Feb, 39(2), 63-73; Kontinen V.K., et al, Electrophysiologic evidence for increased endogenous gabaergic but not glycinergic inhibitory tone in the rat spinal nerve ligation model of neuropathy, *Anesthesiology,* **2001**, Feb, 94(2), 333-9). Various anti-epileptic drugs (AEDs) that stabilize neuronal excitability are effective in neuropathic pain and cluster and migraine headache-associated pain (Delvaux V. and Schoenen J., New generation antiepileptics for facial pain and headache, *Acta Neurol. Belg.,* **2001**, Mar, 101(1), 42-46; Johannessen C.U., Mechanisms of action of valproate: a commentatory, *Neurochem. Int.,* **2000,** Aug-Sep, 37(2-3), 103-110 and Magnus L., Nonepileptic uses of gabapentin, *Epilepsia,* **1999,** 40 Suppl 6, S66-72). Neuropathic pain and cluster and migraine headache-associated pain are widespread conditions that cause suffering.

Substituted phenyl alkyl carbamate compounds have been described in US Patent No. 3,265,728 to Bossinger, et al as useful in treating the central nervous system, having tranquilization, sedation and muscle relaxation properties of the formula:

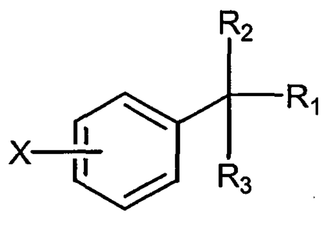

wherein $R_1$ is either carbamate or alkyl carbamate containing from 1 to 3 carbon atoms in the alkyl group; $R_2$ is either hydrogen, hydroxy, alkyl or hydroxy alkyl containing from 1 to 2 carbons; $R_3$ is either hydrogen or alkyl containing from 1 to 2 carbons; and X can be halogen, methyl, methoxy, phenyl, nitro or amino.

A method for inducing calming and muscle relaxation with carbamates has been described in US Patent No. 3,313,692 to Bossinger, et al by administering a compound of the formula:

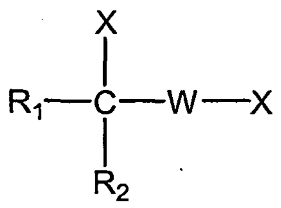

in which W represents an aliphatic radical containing less than 4 carbon atoms, wherein $R_1$ represents an aromatic radical, $R_2$ represents hydrogen or an alkyl radical containing less than 4 carbon atoms, and X represents hydrogen or hydroxy or alkoxy and alkyl radicals containing less than 4 carbon atoms or the radical:

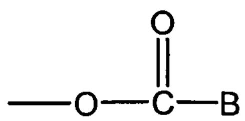

in which B represents an organic amine radical of the group consisting of heterocyclic, ureido and hydrazino radicals and the radical $-N(R_3)_2$ wherein $R_3$ represents hydrogen or an alkyl radical containing less than 4 carbon atoms.

Optically pure forms of halogen substituted 2-phenyl-1,2-ethanediol monocarbamates and dicarbamates have also been described in US Patent No. 6,103,759 to Choi, et al, as effective for treating and preventing central nervous system disorders including convulsions, epilepsy, stroke and muscle spasm; and as useful in the treatment of central nervous system diseases, particularly as anticonvulsants, antiepileptics, neuroprotective agents and centrally acting muscle relaxants, of the formulae:

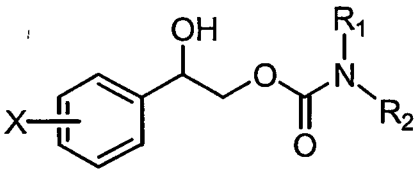
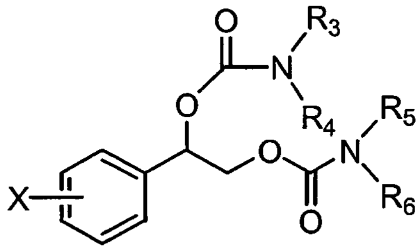

wherein one enantiomer predominates and wherein the phenyl ring is substituted at X with one to five halogen atoms selected from fluorine, chlorine, bromine or iodine atoms and $R_1$, $R_2$, $R_3$, $R_4$, $R_5$ and $R_6$ are each selected from hydrogen and straight or branched alkyl groups with one to four carbons optionally substituted with a phenyl group with substituents selected from the group consisting of hydrogen, halogen, alkyl, alkyloxy, amino, nitro and cyano. Pure enantiomeric forms and enantiomeric mixtures were described wherein one of the enantiomers predominates in the mixture for the compounds represented by the formulae above; preferably one of the enantiomers predominates to the extent of about 90% or greater; and, most preferably, about 98% or greater.

A halogen substituted 2-phenyl-1,2-ethanediol monocarbamate enantiomer of Formula (I) substantially free of other enantiomers or an enantiomeric mixture wherein an enantiomer of Formula (I) predominates has not been previously described as useful for preventing or treating neuropathic pain or cluster and migraine headache-associated pain.

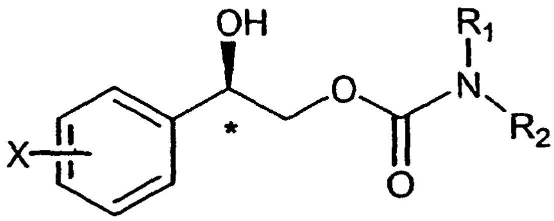

Formula (I)

Recent preclinical studies have revealed previously unrecognized pharmacological properties which suggest that a monocarbamate enantiomer of Formula (I) substantially free of other enantiomers or an enantiomeric mixture wherein an enantiomer of Formula (I) predominates is useful in preventing or treating neuropathic pain and cluster and migraine headache-associated pain. Therefore, it is an object of the present invention to teach a method for use of a monocarbamate enantiomer of Formula (I) substantially free of other enantiomers or an enantiomeric mixture wherein an enantiomer of Formula (I) predominates in preventing or treating neuropathic pain and cluster and migraine headache-associated pain.

The present invention is directed to the use, for the manufacture of a medicament for preventing or treating neuropathic pain and cluster and migraine headache-associated pain, of an enantiomer of Formula (I) free of other enantiomers or an enantiomeric mixture wherein an enantiomer of Formula (I) predominates:

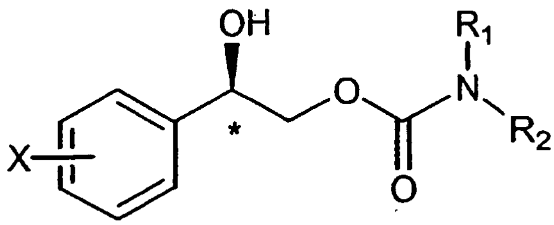

Formula (I)

wherein

phenyl is substituted at X with one to five halogen atoms independently selected from the group consisting of fluorine, chlorine, bromine and iodine; and,

$R_1$ and $R_2$ are independently selected from the group consisting of hydrogen and $C_1$-$C_4$ alkyl; wherein $C_1$-$C_4$ alkyl is optionally substituted with phenyl (wherein phenyl is optionally substituted with substituents independently selected from the group consisting of halogen, $C_1$-$C_4$ alkyl, $C_1$-$C_4$ alkoxy, amino, nitro and cyano).

In one embodiment, the medicament is for preventing or treating neuropathic pain; wherein neuropathic pain results from chronic conditions.

In another embodiment, the medicament is for preventing or treating cluster and migraine headache-associated pain.

The invention uses an enantiomer of Formula (I) free of other enantiomers or an enantiomeric mixture wherein an enantiomer of Formula (I) predominates. For an enantiomeric mixture wherein an enantiomer of Formula (I) predominates, preferably, an enantiomer of Formula (I) predominates to the extent of about 90% or greater. More preferably, an enantiomer of Formula (I) predominates to the extent of about 98% or greater.

The present method includes the use of an enantiomer of Formula (I) free of other enantiomers wherein X is chlorine; preferably, X is substituted at the ortho position of the phenyl ring.

The present method also includes the use of an enantiomer of Formula (I) free of other enantiomers wherein $R_1$ and $R_2$ are preferably selected from hydrogen.

An embodiment of the present method includes the use of an enantiomer of Formula (I) free of other enantiomers

or an enantiomeric mixture wherein an enantiomer of Formula (I) predominates wherein X is chlorine; preferably, X is substituted at the ortho position of the phenyl ring.

The present method also includes the use of an enantiomer of Formula (I) free of other enantiomers or an enantiomeric mixture wherein an enantiomer of Formula (I) predominates wherein $R_1$ and $R_2$ are preferably selected from hydrogen.

An embodiment of the present method includes the use of an enantiomer of Formula (Ia) free of other enantiomers or an enantiomeric mixture wherein an enantiomer of Formula (Ia) predominates:

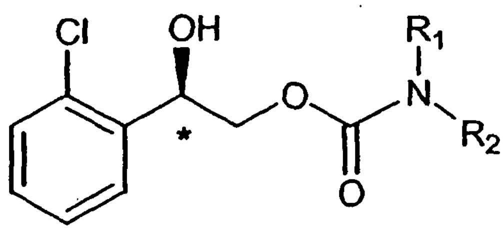

Formula (Ia)

wherein

$R_1$ and $R_2$ are independently selected from the group consisting of hydrogen and $C_1$-$C_4$ alkyl; wherein $C_1$-$C_4$ alkyl is optionally substituted with phenyl (wherein phenyl is optionally substituted with substituents independently selected from the group consisting of halogen, $C_1$-$C_4$ alkyl, $C_1$-$C_4$ alkoxy, amino, nitro and cyano).

The present method also includes the use of an enantiomer of Formula (Ia) free of other enantiomers or an enantiomeric mixture wherein an enantiomer of Formula (Ia) predominates; and, wherein $R_1$ and $R_2$ are preferably selected from hydrogen.

For an enantiomeric mixture wherein an enantiomer of Formula (Ia) predominates, preferably, an enantiomer of Formula (Ia) predominates to the extent of about 90% or greater. More preferably, an enantiomer of Formula (Ia) predominates to the extent of about 98% or greater.

An embodiment of the present method includes the use of an enantiomer of Formula (Ib) free of other enantiomers or an enantiomeric mixture wherein the enantiomer of Formula (Ib) predominates:

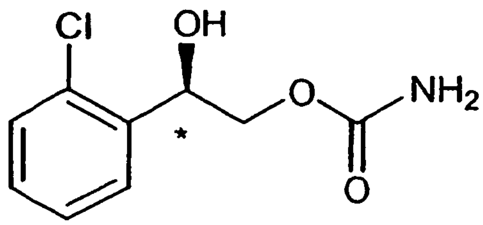

Formula (Ib)

For an enantiomeric mixture wherein the enantiomer of Formula (Ib) predominates, preferably, the enantiomer of Formula (Ib) predominates to the extent of about 90% or greater. More preferably, the enantiomer of Formula (Ib) predominates to the extent of about 98% or greater.

Other crystal forms of an enantiomer of Formula (I) free of other enantiomers may exist and as such are intended to be included in the present invention.

It is apparent to those skilled in the art that the compounds of the invention are present as enantiomers and enantiomeric mixtures thereof. A carbamate enantiomer selected from the group consisting of Formula (I), Formula (Ia) and Formula (Ib) free of other enantiomers contains an asymmetric chiral carbon atom at the benzylic position, which is the aliphatic carbon adjacent to the phenyl ring (represented by the asterisk in the structural formulae).

Compounds of the present invention may be prepared as described in the previously referenced Bossinger ’728 patent Bossinger ’692 patent and Choi ’759 patent.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

In one embodiment of the present invention, the medicament is for preventing or treating neuropathic pain resulting from chronic or debilitating conditions in a subject in need thereof. The chronic or debilitating conditions that lead to neuropathic pain include, but are not limited to, painful diabetic peripheral neuropathy, post-herpetic neuralgia, trigeminal neuralgia, post-stroke pain, multiple sclerosis-associated pain, neuropathies-associated pain such as in idiopathic or post-traumatic neuropathy and mononeuritis, HIV-associated neuropathic pain, cancer-associated neuropathic pain, carpal tunnel-associated neuropathic pain, spinal cord injury-associated pain, complex regional pain syndrome, fibromyalgia-associated neuropathic pain, lumbar and cervical pain, reflex sympathic dystrophy, phantom limb syndrome and other chronic and debilitating condition-associated pain syndromes.

In another embodiment of the present invention, the medicament is for preventing or treating cluster and migraine headache-associated pain in a subject in need thereof. Cluster headache-associated pain is characterized by a series of short-lived attacks on an almost daily basis over a relatively short period of time followed by a pain-free interval. Migraine headache-associated pain is characterized by blinding pain, vomiting, photophobia and recurrence at regular interval; and, includes, but is not limited to, classic migraine headache-associated pain (migraine with aura) and common migraine headache-associated pain (migraine without aura).

In another embodiment of the invention, the medicament is for slowing or delaying the progression of neuropathic pain and cluster and migraine headache-associated pain in a subject in need thereof.

The term "slowing or delaying the progression of neuropathic pain and cluster and migraine headache-associated pain is intended to include minimizing the severity, duration and frequency of the clinical manifestations associated with neuropathic pain and cluster and migraine headache-associated pain in a subject.

An enantiomer of Formula (I) free of other enantiomers or an enantiomeric mixture wherein an enantiomer of Formula (I) predominates may be administered in a pharmaceutical composition comprising a pharmaceutically acceptable carrier.

The enantiomer of Formula (I) free of other enantiomers or enantiomeric mixture wherein an enantiomer of Formula (I) predominates may be used in combination with one or more agents useful in preventing or treating neuropathic pain and cluster and migraine headache-associated pain.

An enantiomer of Formula (I) free of other enantiomers or enantiomeric mixture wherein an enantiomer of Formula (I) predominates or pharmaceutical composition thereof may be administered by any conventional route of administration including, but not limited to oral, pulmonary, intraperitoneal (ip), intravenous (iv), intramuscular (im), subcutaneous (sc), transdermal, buccal, nasal, sublingual, ocular, rectal and vaginal. In addition, administration directly to the nervous system may include, and are not limited to, intracerebral, intraventricular, intracerebroventricular, intrathecal, intracisternal, intraspinal or peri-spinal routes of administration by delivery via intracranial or intravertebral needles or catheters with or without pump devices. It will be readily apparent to those skilled in the art that any dose or frequency of administration that provides the therapeutic effect described herein is suitable for use in the present invention.

The therapeutically effective amount of an enantiomer of Formula (I) free of other enantiomers or enantiomeric mixture wherein an enantiomer of Formula (I) predominates or pharmaceutical composition thereof may be from about 0.01 mg/Kg/dose to about 100 mg/Kg/dose. Preferably, the therapeutically effective amount may be from about 0.01 mg/Kg/dose to about 25 mg/Kg/dose. More preferably, the therapeutically effective amount may be from about 0.01 mg/Kg/dose to about 10 mg/Kg/dose. Most preferably, the therapeutically effective amount may be from about 0.01 mg/Kg/dose to about 5 mg/Kg/dose. Therefore, the therapeutically effective amount of the active ingredient contained per dosage unit (e.g., tablet, capsule, powder, injection, suppository, teaspoonful and the like) as described herein may be from about 1 mg/day to about 7000 mg/day for a subject, for example, having an average weight of 70 Kg.

The dosages, however, may be varied depending upon the requirement of the subjects (including factors associated with the particular subject being treated, including subject age, weight and diet, strength of the preparation, the advancement of the disease condition and the mode and time of administration).

Optimal dosages to be administered may be readily determined by those skilled in the art and will result in the need to adjust the dose to an appropriate therapeutic level. The use of either daily administration or post-periodic dosing may be employed. Preferably, an enantiomer of Formula (I) free of other enantiomers or enantiomeric mixture wherein an enantiomer of Formula (I) predominates or pharmaceutical composition thereof is administered orally or parenterally. More preferably, an enantiomer of Formula (I) free of other enantiomers or enantiomeric mixture wherein an enantiomer of Formula (I) predominates or pharmaceutical composition thereof is administered orally.

An enantiomer of Formula (I) free of other enantiomers or enantiomeric mixture wherein an enantiomer of Formula (I) predominates or pharmaceutical composition thereof described herein may be administered separately, at

different times during the course of therapy or concurrently in divided combination or single combination forms. Advantageously, an enantiomer of Formula (I) free of other enantiomers or enantiomeric mixture wherein an enantiomer of Formula (I) predominates or pharmaceutical composition thereof may be administered in a single daily dose or the total daily dosage may be administered via continuous delivery or in divided doses of two, three or four times daily. The instant invention is therefore to be understood as embracing all such regimes of simultaneous or alternating treatment and the term "administering" is to be interpreted accordingly.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system (preferably, an animal; more preferably, a mammal; most preferably, a human) that is being sought by a researcher, veterinarian, medical doctor, or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

To prepare a pharmaceutical composition of the present invention, an enantiomer of Formula (I) free of other enantiomers or enantiomeric mixture wherein an enantiomer of Formula (I) predominates as the active ingredient is intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

Preferably a pharmaceutical composition is in a unit dosage form such as a tablet, pill, capsule, caplet, gelcap, lozenge, granule, powder, sterile parenteral solution or suspension, metered aerosol or liquid spray, drop, ampoule, autoinjector device or suppository for administration by oral, intranasal, sublingual, intraocular, transdermal, parenteral, rectal, vaginal, inhalation or insufflation means. Alternatively, the composition may be presented in a form suitable for once-weekly or once-monthly administration or may be adapted to provide a preparation for intramuscular injection.

In preparing a pharmaceutical composition having a solid dosage form for oral administration, such as a tablet, pill, capsule, caplet, gelcap, lozenge, granule or powder (each including immediate release, timed release and sustained release formulations), suitable carriers and additives include but are not limited to diluents, granulating agents, lubricants, binders, glidants, disintegrating agents and the like. If desired, tablets may be sugar coated, gelatin coated, film coated or enteric coated by standard techniques.

For preparing a solid dosage form, the principal active ingredient is mixed with a pharmaceutical carrier (e.g. conventional tableting ingredients such as diluents, binders, adhesives, disintegrants, lubricants, antiadherents and glidants). Sweeteners and flavorants may be added to chewable solid dosage forms to improve the palatability of the oral dosage form. Additionally, colorants and coatings may be added or applied to the solid dosage form for ease of identification of the drug or for aesthetic purposes. These carriers are formulated with the pharmaceutical active to provide an accurate, appropriate dose of the pharmaceutical active with a therapeutic release profile.

In preparing a pharmaceutical composition having a liquid dosage form for oral, topical and parenteral administration, any of the usual pharmaceutical media or excipients may be employed. Thus, for liquid unit dosage forms, such as suspensions (i.e. colloids, emulsions and dispersions) and solutions, suitable carriers and additives include but are not limited to pharmaceutically acceptable wetting agents, dispersants, flocculation agents, thickeners, pH control agents (i.e. buffers), osmotic agents, coloring agents, flavors, fragrances, preservatives (i.e. to control microbial growth, etc.) and a liquid vehicle may be employed. Not all of the components listed above will be required for each liquid dosage form. The liquid forms in which the novel compositions of the present invention may be incorporated for administration orally or by injection include, but are not limited to aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles.

## Biological Experimental Examples

The activity of an enantiomer of Formula (I) substantially free of other enantiomers for use in the treatment of neuropathic pain was evaluated in the following experimental examples and is intended to be a way of illustrating but not limiting the invention.

The procedure used to test the antiallodynic activity of an enantiomer of Formula (I) substantially free of other enantiomers was the procedure for the measurement of allodynia found in the Chung Model (Kim S.H. and Chung J.M., An Experimental Model for Peripheral Neuropathy Produced by Segmental Spinal Nerve Ligation in the Rat, *Pain,* **1992**, 50, 355-363).

*Evaluation of Antiallodynic Activity (Manually Applied Von Frey Probes) Animals*

Pathogen-free, male albino Sprague-Dawley rats, 200 g, were purchased from Harlan Industries (Indianapolis, IN) and maintained on a 12-h light/dark cycle (lights on at 06:00 h) in a climate-controlled room with food and water available *ad libitum* up to the time of the testing and food withdrawn 18 hr prior to testing.

*Surgical procedure and measurement of allodynia*

The rats were anesthetized with isoflurane inhalant anesthesia. The left lumbar spinal nerve at the level of L5 was tightly ligated (4-0 silk suture) distal to the dorsal root ganglion and prior to entrance into the sciatic nerve, as described by Kim and Chung. The incisions were closed and the rats were allowed to recover under conditions described above. This procedure results in mechanical allodynia in the left hind paw. The sham operation, when performed, consisted of a similar surgical procedure lacking only the final ligation of the spinal nerve. Mechanical (tactile) allodynia was assessed by recording the pressure at which the affected paw (ipsilateral to the site of nerve injury) was withdrawn from graded stimuli (von Frey filaments ranging from 4.0 to 148.1 mN) applied by hand perpendicularly to the plantar surface of the paw (between the footpads) through wire-mesh observation cages. A paw withdrawal threshold (PWT) was determined by sequentially increasing and decreasing the stimulus strength and analyzing withdrawal data using a Dixon non-parametric test, as described by Chaplan et al (Chaplan S.R., Bach F.W., Pogrel J.W., Chung J.M. and Yaksh T.L., Quantitative Assessment of Tactile Allodynia in the Rat Paw, *J Neurosci Meth,* **1994**, 53, 55-63). Normal rats, sham operated rats, and the contralateral paw of L5 ligated rats withstand at least 148.1 mN (equivalent to 15 g) of pressure without responding. Spinal nerve ligated rats respond to as little as 4.0 mN (equivalent to 0.41 g) of pressure on the affected paw. Rats were included in the study only if they did not exhibit motor dysfunction *(e.g.,* paw dragging or dropping) and their PWT was below 39.2 mN (equivalent to 4.0 g). The PWT was used to calculate the % maximal possible effect (% MPE) according to the formula:

$$\% \text{ MPE} = 100 \times (\text{PWT} - \text{CT}) / (\text{CO} - \text{CT}).$$

*Data Analysis*

As summarized in Table 1 below, the enantiomer of Formula (Ib) substantially free of other enantiomers was screened for antiallodynic activity in the Chung model of neuropathic pain at a dose of 30 and 100 mg/kg, po, with responses being measured at 0.5, 1, 2 and 4 hr post dosing; responses returned to baseline by one hour. Data shown are from testing at 30 min after oral dosing, the time of peak antiallodynic effect, with N=5 animals per dose.

**Table 1**
**Antiallodynic Effect Assessed with Manually Applied Von Frey Probes**

| Dose (mg/Kg) | % Maximum Possible Effect | n |
|---|---|---|
| 30 | 11 | 5 |
| 100 | 28 | 5 |

## Claims

1. The use, for the manufacture of a medicament for preventing or treating neuropathic pain and cluster and migraine headache-associated pain, of an enantiomer of Formula (I) free of other enantiomers or an enantiomeric mixture wherein an enantiomer of Formula (I) predominates:

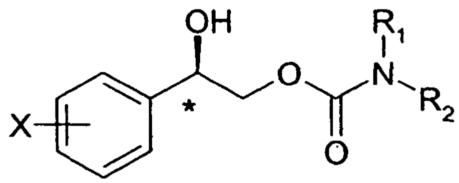

Formula (I)

wherein

phenyl is substituted at X with one to five halogen atoms independently selected from the group consisting of fluorine, chlorine, bromine and iodine; and
$R_1$ and $R_2$ are independently selected from the group consisting of hydrogen and $C_1$-$C_4$ alkyl; wherein $C_1$-$C_4$ alkyl is optionally substituted with phenyl, wherein phenyl is optionally substituted with substituents independently selected from the group consisting of halogen, $C_1$-$C_4$ alkyl, $C_1$-$C_4$ alkoxy, amino, nitro and cyano.

**2.** The use according to claim 1 wherein said medicament is for preventing or treating neuropathic pain.

**3.** The use according to claim 1 wherein said medicament is for preventing or treating cluster and migraine headache-associated pain.

**4.** The use according to claim 1 wherein said medicament is for slowing or delaying the progression of neuropathic pain and cluster and migraine headache associated pain.

**5.** The use of claim 1 wherein X is chlorine.

**6.** The use of claim 1 wherein X is substituted at the ortho position of the phenyl ring.

**7.** The use of claim 1 wherein $R_1$ and $R_2$ are hydrogen.

**8.** The use of any of claims 1 to 4 wherein the enantiomer of Formula (I) is an enantiomer of Formula (Ib) free of other enantiomers or an enantiomeric mixture wherein the enantiomer of Formula (Ib) predominates:

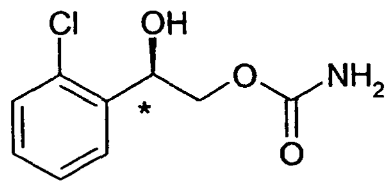

Formula (Ib)

**9.** The use of claim 8 wherein the enantiomer of Formula (Ib) predominates to the extent of 90% or greater.

**10.** The use of claim 8 wherein the enantiomer of Formula (Ib) predominates to the extent of 98% or greater.

**11.** The use of claim 2 wherein neuropathic pain results from a condition selected from the group consisting of painful diabetic peripheral neuropathy, post-herpetic neuralgia, trigeminal neuralgia, post-stroke pain, multiple sclerosis-associated pain, neuropathies-associated pain such as in idiopathic or post-traumatic neuropathy and mononeuritis, HIV-associated neuropathic pain, cancer-associated neuropathic pain, carpal tunnel-associated neuropathic pain, spinal cord injury-associated pain, complex regional pain syndrome, fibromyalgiaassociated neuropathic pain, lumbar and cervical pain, reflex sympathetic dystrophy, and phantom limb syndrome.

**12.** The use of claim 1 or claim 8 wherein the enantiomer of Formula (I) is for administration in an amount from 0.01 mg/Kg/dose to 100 mg/Kg/dose.

## Patentansprüche

**1.** Verwendung, zur Herstellung eines Arzneimittels zur Prävention oder Behandlung von neuropathischen Schmerzen und migräneartigen Kopfschmerzen, eines Enantiomers von Formel (I), das frei von anderen Enantiomeren ist, oder einer Enantiomerenmischung, in der ein Enantiomer von Formel (I) überwiegt:

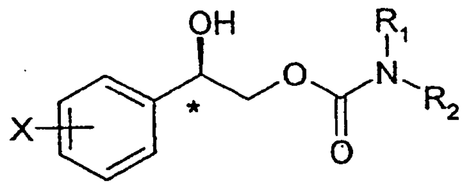

Formel (I)

worin

Phenyl bei X mit einem bis fünf Halogenatomen substituiert ist, die unabhängig ausgewählt sind aus der Gruppe, bestehend aus Fluor, Chlor, Brom und Iod; und
$R_1$ und $R_2$ unabhängig ausgewählt sind aus der Gruppe, bestehend aus Wasserstoff und $C_1$-$C_4$-Alkyl; wobei $C_1$-$C_4$-Alkyl fakultativ substituiert ist mit Phenyl, wobei Phenyl fakultativ substituiert ist mit Substituenten, die unabhängig ausgewählt sind aus der Gruppe, bestehend aus Halogen, $C_1$-$C_4$-Alkyl, $C_1$-$C_4$-Alkoxy, Amino, Nitro und Cyano.

**2.** Verwendung nach Anspruch 1, **dadurch gekennzeichnet, daß** besagtes Arzneimittel für die Prävention oder Behandlung neuropathischer Schmerzen ist.

**3.** Verwendung nach Anspruch 1, **dadurch gekennzeichnet, daß** besagtes Arzneimittel für die Prävention oder Behandlung von migräneartigen Kopfschmerzen ist.

**4.** Verwendung nach Anspruch 1, **dadurch gekennzeichnet, daß** besagtes Arzneimittel zur Verlangsamung oder Verzögerung des Fortschreitens von neuropathischen Schmerzen und migräneartigen Kopfschmerzen ist.

**5.** Verwendung nach Anspruch 1, **dadurch gekennzeichnet, daß** X Chlor ist.

**6.** Verwendung nach Anspruch 1, **dadurch gekennzeichnet, daß** X an der ortho-Position des Phenylrings substituiert ist.

**7.** Verwendung nach Anspruch 1, **dadurch gekennzeichnet, daß** $R_1$ und $R_2$ Wasserstoff sind.

**8.** Verwendung nach einem der Ansprüche 1 bis 4, **dadurch gekennzeichnet, daß** das Enantiomer von Formel (I) ein Enantiomer von Formel (Ib), das frei von anderen Enantiomeren ist, oder eine Enantiomerenmischung ist, in der das Enantiomer von Formel (Ib) überwiegt:

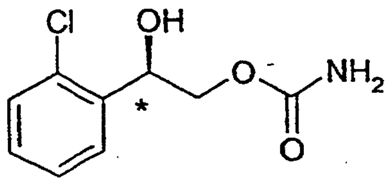

Formel (Ib)

9. Verwendung nach Anspruch 8, **dadurch gekennzeichnet, daß** das Enantiomer von Formel (Ib) im Umfang von 90% oder mehr überwiegt.

10. Verwendung nach Anspruch 8, **dadurch gekennzeichnet, daß** das Enantiomer von Formel (Ib) im Umfang von 98% oder mehr überwiegt.

11. Verwendung nach Anspruch 2, **dadurch gekennzeichnet, daß** neuropathische Schmerzen aus einem Zustand resultieren, der ausgewählt ist aus der Gruppe, bestehend aus schmerzhafter diabetischer peripherer Neuropathie, nach einem Herpes auftretender Neuralgie, Trigeminal-Neuralgie, nach einem Schlaganfall auftretenden Schmerzen, mit multipler Sklerose assoziierten Schmerzen, mit Neuropathien assoziierten Schmerzen, wie etwa bei idiopathischer oder posttraumatischer Neuropathie und Mononeuritis, mit HIV-assoziierten neuropathischen Schmerzen, mit Krebs assoziierten neuropatischen Schmerzen, mit dem Karpaltunnel assoziierten neuropathischen Schmerzen, mit einer Rückenmarkverletzung assoziierten Schmerzen, komplexem regionalen Schmerzsyndrom, mit Fibromyalgie assoziierten neuropathischen Schmerzen, Lumbal- und Cervikalschmerzen, reflexsympathischer Dystrophie und Phantomgliedsyndrom.

12. Verwendung nach Anspruch 1 oder Anspruch 8, **dadurch gekennzeichnet, daß** das Enantiomer von Formel (I) für Verabreichung in einer Menge von 0,01 mg/kg/Dosis bis 100 mg/kg/Dosis ist.

**Revendications**

1. Utilisation, pour la fabrication d'un médicament pour la prévention ou le traitement de la douleur neuropathique et de la douleur associée à la céphalite vasculaire de Horton et à la migraine, d'un énantiomère de Formule (I) sans autres énantiomères ou d'un mélange d'énantiomères où prédomine un énantiomère de Formule (I) :

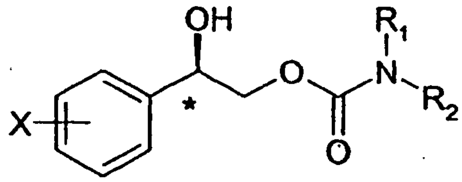

Formule (I)

où

phényle est substitué en X avec un à cinq atomes d'halogène indépendamment sélectionnés dans le groupe consistant en fluor, chlore, brome et iode ; et
$R_1$ et $R_2$ sont indépendamment sélectionnés dans le groupe consistant en hydrogène et alkyle $C_1$-$C_4$ ; où alkyle $C_1$-$C_4$ est facultativement substitué par phényle où phényle est facultativement substitué par des substituants

indépendamment sélectionnés dans le groupe consistant en halogène, alkyle $C_1$-$C_4$, alcoxy $C_1$-$C_4$, amine, nitro et cyano.

**2.** Utilisation selon la revendication 1, où ledit médicament est pour la prévention ou le traitement de la douleur neuropathique.

**3.** Utilisation selon la revendication 1, où ledit médicament est pour la prévention ou le traitement de la douleur associée à la céphalée vasculaire de Horton et à la migraine.

**4.** Utilisation selon la revendication 1, où ledit médicament est pour ralentir ou retarder la progression de la douleur neuropathique et de la douleur associée à la céphalée vasculaire de Horton et à la migraine.

**5.** Utilisation de la revendication 1, où X est chlore.

**6.** Utilisation de la revendication 1 où X est substitué à la position ortho du cycle phényle.

**7.** Utilisation de la revendication 1, où $R_1$ et $R_2$ sont hydrogène.

**8.** Utilisation de l'une quelconque des revendications 1 à 4, où l'énantiomère de Formule (I) est un énantiomère de Formule (1b) sans autres énantiomères ou bien un mélange énantiomère où l'énantiomère de Formule (Ib) prédomine :

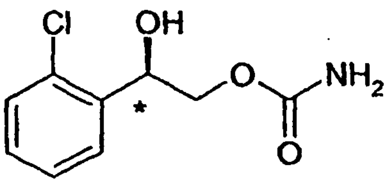

Formule (Ib)

**9.** Utilisation de la revendication 8, où l'énantiomère de Formule (1b) prédomine jusqu'à 90% ou plus.

**10.** Utilisation de la revendication 8, où l'énantiomère de Formule (Ib) prédomine jusqu'à 98% ou plus.

**11.** Utilisation de la revendication 2, où la douleur neuropathique résulte d'une condition sélectionnée dans le groupe consistant en neuropathie périphérique diabétique, douleureuse névralgie post-herpétique, névralgie trigéminale, douleur après congestion, douleur associée à la sclérose en plaques, douleur associée à des neuropathies comme neuropathie iodopathie ou post-traumatique et la mononeurite, douleur neuropathique associée à VIH, douleur neuropathique associée au cancer, douleur neuropathique associée au tunnel carpien, douleur associée à une lésion de la moelle épinière, syndrome de douleur régionale complexe, douleur neuropathique associée à une fibromyalgie, douleur lombaire et cervicale, dystrophie sympathique reflexe et syndrome du membre fantôme.

**12.** Utilisation de la revendication 1 ou de la revendication 8, où l'énantiomère de Formule (I) est pour administration d'une quantité de 0,01 mg/kg/dose à 100 mg/kg/dose.